# EUROPÄISCHE PATENTSCHRIFT

(11) **EP 1 424 582 B1**
(45) Veröffentlichungstag und Bekanntmachung des Hinweises auf die Patenterteilung: **01.03.2006**
(21) Anmeldenummer: 03026041.8
(22) Anmeldetag: 12.11.2003
(51) Int. Cl.: G02B 21/22

(54) **Stereomikroskop**
Stereomicroscope
Stéréomicroscope

(30) Priorität: 29.11.2002 DE 10255961
(43) Veröffentlichungstag der Anmeldung: 02.06.2004
(73) Patentinhaber: Leica Microsystems (Schweiz) AG, 9435 Heerbrugg (CH)
(72) Erfinder: Sander, Ulrich, Dr., 9445 Rebstein (CH)
(74) Vertreter: Hössle Kudlek & Partner

(56) Entgegenhaltungen:
- DE-A- 3 333 471
- DE-A- 4 331 635
- US-A- 5 898 518
- US-A1- 2001 010 592

## Beschreibung

Die vorliegende Erfindung betrifft ein Stereomikroskop nach dem Oberbegriff des Patentanspruchs 1.

In der Ophthalmologie und Neurochirurgie eingesetzte Operationsmikroskope bieten die Möglichkeit, dass sowohl ein Hauptoperateur als auch ein Assistent das gleiche Operationsfeld einsehen können.

Ein derartiges ophthalmologisches Operationsmikroskop ist beispielsweise aus der DE 43 31 635 C2 bekannt. Das dort beschriebene Operationsmikroskop weist je einen Binokulartubus für einen Haupt- und einen Mitbeobachter sowie einen Strahlteiler 4, der das Objektlicht auf den Haupt- und Mitbeobachter aufteilt, auf. Als nachteilig bei diesem Mikroskop wird angesehen, dass es relativ hoch baut, da die vollständige Vergrößerungsoptik für den Hauptbeobachter im wesentlichen vertikal angeordnet ist.

Aus der DE 33 33 471 C2 ist ein Stereomikroskop für die Anwendung in der Neurochirurgie zur Simultänbeobachtung für einen ersten und einen zweiten Beobachter bekannt. Hierbei erfolgt eine Aufteilung der Strahlengänge für den ersten und zweiten Beobachter mittels einer Teilungsplatte, wodurch ein Lichtintensitätsverlust in Kauf genommen werden muß. Zusätzlich wird der freie Arbeitsabstand zwischen Objektiv und Objekt stark reduziert.

Ein ähnliches Mikroskop ist schließlich aus der US 5,898,518 bekannt.

Derartige Operationsmikroskope haben in der Praxis eine Anzahl grundlegender Anforderungen zu erfüllen.

Eine Anforderung besteht darin, dass die Bauhöhe des Mikroskops aus ergonomischen Gründen so gering wie möglich gehalten wird. Ferner wird gefordert, dass der Assistenteneinblick schnell und ohne Umbauarbeiten von der rechten zur linken Seite des Mikroskops (oder umgekehrt) verschwenkt werden kann, und dass durch Zubehör (optische bzw. optisch-mechanische Zusatzkomponenten), welches nur für bestimmte Operationstechniken benötigt wird, weder die Bildqualität noch die Bauhöhe negativ beeinflusst wird. Ferner sollen sowohl der Hauptbeobachter als auch der Assistent die Möglichkeit haben, den sogenannten Red-Reflex in gleicher Güte zu beobachten.

Bei herkömmlichen Mikroskopen werden diese Anforderungen nur teilweise erfüllt.

Bei dem Operationsmikroskop M840/M841 der Anmelderin ist beispielsweise gewährleistet, dass der Assistent und der Hauptoperateur tatsächlich das gleiche Blickfeld haben.

Dies wird dadurch erreicht, dass die assistentische Beobachtungseinrichtung oberhalb des Vergrößerungssystems angeordnet ist, und als Vergrößerungssystem ein Zoom-System verwendet wird, dass aus vier identischen monoskopischen Vergrößerungssystemen aufgebaut ist. Dabei bilden jeweils zwei von den vier zueinander parallel liegenden Systemen das stereoskopische Vergrößerungssystem für den Hauptbeobachter. Auf der Verbindungsachse dieser Systeme senkrecht liegende weitere Systeme bzw. Kanäle stellen hierbei das stereoskopische Vergrößerungssystem für den Assistenten dar.

Aus der US 2001/001 05 92 A1, dem nächsten Stand der Technik, ist ein in der Neurochirurgie einsetzbares Mikroskop bekannt, welches ein Objektivsystem, ein Zoom-System und ein Okular-System aufweist. Hierbei ist das Objektivsystem im wesentlichen vertikal angeordnet, während das aus zwei Einzelsystemen bzw. optischen Kanälen bestehende Zoom-System horizontal angeordnet ist. Die wesentliche Neuerung bei dem dort beschriebenen Mikroskop liegt darin begründet, dass die Achse des Zoom-Systems senkrecht zu der Achse des Hauptobjektivs liegt. Das Zoom-System besteht aus zwei identischen Vergrößerungskanälen, deren Achsen parallel zueinander verlaufen, womit die stereoskopische Betrachtung eines Objektes gewährleistet ist. Als nachteilig bei dem dort beschriebenen Mikroskop wird empfunden, dass aufgrund der Verwendung eines halbdurchlässigen Strahlenteilers zur räumlichen Trennung eines Assistenten-Strahlengangs von einem Hauptoperateur-Strahlengang relativ starke Lichtverluste nicht vermieden werden können. Da der Strahlenteiler des Assistentenmikroskops auch vom Beleuchtungsstrahlengang durchsetzt wird, entstehen dort äußerst schwierig zu beseitigende Reflexionen. Dieser Strahlenteiler wird konvergent vom Beobachtungsstrahlengang durchsetzt und führt damit zu schwer zu beseitigenden Bildfehlern. Mit dieser Anordnung des Strahlenteilers wird zusätzlich ein Astigmatismus in Kauf genommen, der von der relativen Orientierung des Assistentenmikroskops zum Hauptmikroskop abhängt.

Die vorliegende Erfindung hat zum Ziel, ein Stereomikroskop mit einfacher Handhabbarkeit und geringer Bauhöhe zur Verfügung zu stellen.

Dieses Ziel wird erreicht mit einem Stereomikroskop mit den Merkmalen des Patentanspruchs 1.

Mit der Maßnahme, auf der Achse des Zoom-Systems und/oder der wenigstens einen hierzu im wesentlichen parallel verlaufenden Achse wenigstens ein optisches Element zur Verlängerung des Strahlenganges des wenigstens einen Beobachtungsstrahlenbüschels entlang der optischen Achse des Zoom-Systems bzw. der hierzu parallelen Achse vorzusehen, kann die Leistungsfähigkeit bzw. vielseitige Einsetzbarkeit eines Stereomikroskops in einfacher Weise verbessert werden, ohne in ergonomisch ungünstiger Weise die Bauhöhe des Mikroskops zu beeinflussen. Dazu sind in die in beliebiger Weise verlängerbaren Strahlengänge wahlweise optische Zusatzkomponenten einsetzbar, welche keinen Einfluss auf die Bauhöhe des Mikroskops haben. Die Verlängerung der Strahlengänge führt lediglich zu einer im wesentlichen vom Beobachter weg gerichteten horizontalen Verlängerung des Stereomikroskops, welche für den Beobachter, insbesondere für einen Operateur, mit keinerlei ergonomischen Nachteilen verbunden ist, sondern sogar zu einer besseren Schwerpunktslage führt. Mit der Verlängerung der Strahlengänge ist es insgesamt in einfacher Weise möglich, zusätzliche Baugruppen/Module in die Strahlengänge einzusetzen, ohne dass Vignettierungs- bzw. Feldbeschnitteffekte auftreten.

Erfindungsgemäß erzeugt das wenigstens eine optische Element zur Verlängerung des Strahlengangs Zwischenabbildungen eines beobachteten Objektes. Mittels derartiger Zwischenabbildungen ist insgesamt eine mehrstufige Abbildung eines zu beobachtenden Objektes realisierbar, wodurch insgesamt die jeweiligen Strahlengänge in einfacher Weise verlängerbar sind. Eine "mehrstufige Abbildung" bedeutet insbesondere die Schaffung einer Anzahl von Zwischenbildern. Der Begriff "Verlängerung des Strahlenganges" umfasst sowohl die Verlängerung der mechanischen als auch optischen Weglänge. Diese Verlängerung kann kontinuierlich, mechanisch durch eine "Auszugsverlängerung" realisiert werden. Hierbei sind für die Auszugsverlängerung verwendete Linsensysteme derart ausgelegt, dass eine mechanische Wegverlängerung ohne Bildqualitätsverlust erreicht wird. Zweckmäßigerweise werden in den Zwischenabbildungsebenen oder in deren Nähe Feldlinsen eingesetzt, welche die Strahlenbüschel in geeigneter Weise auf ein nachfolgendes optisches Element lenken. Somit bedeutet der verwendete Begriff "Auszugsverlängerung" insbesondere eine variable kontinuierliche mechanische Verlängerung der Strahlengänge ohne Veränderung der optischen Eigenschaften.

Gemäß einer besonders bevorzugten Ausführungsform des erfindungsgemäßen Stereomikroskops ist auf der Achse des Zoom-Systems und/oder der wenigstens einen hierzu parallel verlaufenden Achse wenigstens eine optisch-mechanische Komponente, insbesondere eine Dateneinspiegelungseinrichtung, eine Invertereinrichtung, beispielsweise eine SDI-Einrichtung, eine Laser-Shuttereinrichtung oder ein optischer Teiler vorgesehen. Derartige Bauteile sind nun erfindungsgemäß in einfacher Weise in ein Stereomikroskop, insbesondere ein ophthalmologisches Stereomikroskop oder eines für die Neurochirurgie, integrierbar, ohne die Bauhöhe des Mikroskops in ergonomisch ungünstiger Weise zu beeinflus-Mikroskops in ergonomisch ungünstiger Weise zu beeinflussen. Bei herkömmlichen ophthalmologischen Stereomikroskopen konnten oftmals derartige Bauteile nur im Sinne eines Kompromisses eingesetzt werden, da deren Einbau zu einer ergonomisch nicht handhabbaren Beabstandung zwischen zu dem beobachtenden (und gegebenenfalls zu operierenden) Objekt und der Beobachtungsachse des Operateurs geführt hätte und die Bildqualität deutlich reduziert wurde. Die genannten Komponenten sind an sich bekannt, wobei darauf hingewiesen sei, dass es sich bei einer SDI-Einrichtung (Stereoskopischer-Diagonal-Inverter) um ein optisches Element zur Beobachtung in den hinteren Augenabschnitten während der Operation handelt.

Zweckmäßigerweise verlaufen die Lichtrichtungen des wenigstens einen Beobachtungsstrahlenbüschels entlang der Achse des Zoom-Systems und der hierzu parallel verlaufenden Achse entgegengesetzt zueinander. Mit dieser Ausrichtung können zunächst die Beobachtungsstrahlenbüschel vom Beobachter bzw. Operateur weggeführt werden, und anschließend, nach entsprechender Umlenkung, zurück zu einem Binokulartubus mit Okularen des Beobachters geführt werden.

Durch entsprechende Anordnung der optischen Zusatzkomponenten auf den jeweiligen Achsen kann so auch die Baugröße des Mikroskops in Richtung der optischen Achse des Zoom-Systems optimiert werden.

Gemäß einer besonders bevorzugten Ausführungsform des erfindungsgemäßen Stereomikroskops weist das Zoom-System wenigstens drei, insbesondere vier Vergrößerungs- bzw. Beobachtungskanäle auf. Mittels einer derartigen Ausbildung ist es in besonders lichtökonomischer Weise möglich, Beobachtungsachsen für einen Hauptbeobachter bzw. -operateur und einen Assistenten zu definieren. Hierbei ist es auch möglich, ein ophthalmologisches Operationsmikroskop mit der Möglichkeit einer stereoskopischen Betrachtung für einen Hauptoperateur und lediglich einer monoskopischen Betrachtung durch einen Assistenten vorzusehen. Dadurch, dass hierfür in dem Zoom-System drei Vergrößerungs- bzw. Beobachtungskanäle vorgesehen sind, ist eine räumliche Trennung von Hauptoperateur-Strahlengang und Assistenten-Strahlengang zur Definition jeweiliger Beobachtungsachsen in einfacher Weise ohne die Notwendigkeit der Verwendung von halbdurchlässigen Strahlenteilern möglich. Aufgrund der horizontal verlaufenden Vergrößerungs- bzw. Beobachtungskanäle ist hierbei gleichzeitig eine sehr kleine Bauhöhe des Mikroskops realisierbar, welche, wie erwähnt aus ergonomischen Gründen sehr günstig ist. Es ist ebenfalls denkbar, den dritten Vergrößerungskanal für den Anschluss einer Dokumentationseinrichtung, beispielsweise einer Kamera, zu nutzen. Bei der Ausführungsform mit vier Beobachtungskanälen ist sowohl durch den Hauptbeobachter als auch durch den Assistenten eine stereoskopische Beobachtung des Objektes möglich. Das Vorsehen von vier Vergrößerungskanälen im Rahmen des Zoom-Systems stellt eine bevorzugte Ausführungsform des erfindungsgemäßen Stereomikroskops dar, da hierbei sowohl für den Hauptoperateur als auch für den Assistenten ein geringer vertikaler Abstand zwischen jeweiliger Beobachtungsachse und Objekt realisiert ist, wobei - wie erwähnt - eine besonders günstige Licht-Ausbeute gewährleistet ist. Es ist ebenfalls denkbar, in dem Zoom-System mehr als vier, beispielsweise sechs oder acht Vergrößerungskanäle, oder eine ungerade Anzahl, vorzusehen.

Es ist bevorzugt, dass die Achse des Objektivs im wesentlichen vertikal und die Achse des Zoom-Systems im wesentlichen horizontal verläuft. Mit dieser Anordnung der Achsen ist das erfindungsgemäße Stereomikroskop ergonomisch optimierbar.

Gemäß einer weiteren bevorzugten Ausführungsform des erfindungsgemäßen Stereomikroskops ist zwischen dem Hauptobjektiv und dem Zoom-System ein Strahlenteiler angeordnet. Mittels eines derartigen Strahlenteilers ist ein weiterer Strahlengang aus dem Hauptstrahlengang teilbar, mittels dessen das zu beobachtende Objekt von einem weiteren Assistenten beobachtet werden kann. Es ist insbesondere möglich, dass der Strahlenteiler gemeinsam mit dem Assistentenmikroskop um die optische Achse des Hauptobjektivs kontinuierlich drehbar ist und damit in jeder Drehlage optisch benutzbar ist. Unter kontinuierlicher Drehbarkeit wird hierbei eine Rotation um die optische Achse des Hauptobjektivs verstanden, die einen beliebigen Drehwinkel um die Achse stufenlos und/oder in Stufen ermöglicht. Diese Maßnahme trägt somit zu einer besonders flexibel handhabbaren Positionierung des Assistentenmikroskops bei. Zweckmäßigerweise ist das Assistentenmikroskop an einer mechanischen Trennstelle vom Hauptmikroskop abtrennbar und/oder entfernbar. Mit dieser Maßnahme kann die Handhabbarkeit des Mikroskops weiter verbessert werden, indem dass Assistentenmikroskop dann entfernbar ist, wenn es nicht benötigt wird.

Zweckmäßigerweise ist die Dateneinspiegelungseinrichtung derart ausgebildet, dass eine Dateneinspiegelung vor und/oder hinter dem Zoom-System möglich ist. Mit dieser Maßnahme kann gewährleistet werden, dass beispielsweise eingespiegelte Daten, wie etwa Vergrößerungs- oder Größenangaben von einer konkreten Zoombetätigung unbeeinflusst bleiben, indem diese Daten hinter dem Zoom-System in den optischen Strahlengang eingespiegelt werden. Ist es hingegen erforderlich, beispielsweise ein Referenzbild mit einem mikroskopisch zu beobachtenden Bild zu vergleichen, erfolgt die Einspiegelung des Referenzbildes zweckmäßigerweise vor dem Zoom-System, so dass mittels des Zoom-Systems eine gleichzeitige Vergrößerung von zu beobachtendem Bild und Referenzbild möglich ist.

Die Erfindung wird nun anhand der beigefügten Zeichnung weiter erläutert. In dieser zeigt
- Figur 1: in schematischer seitlicher Ansicht den Gesamtaufbau einer bevorzugten Ausführungsform des erfindungsgemäßen Stereomikroskops, und
- Figur 2: eine Querschnittsansicht einer bevorzugten Ausführungsform eines erfindungsgemäß verwendbaren Zoom-Systems bzw. Pankraten.

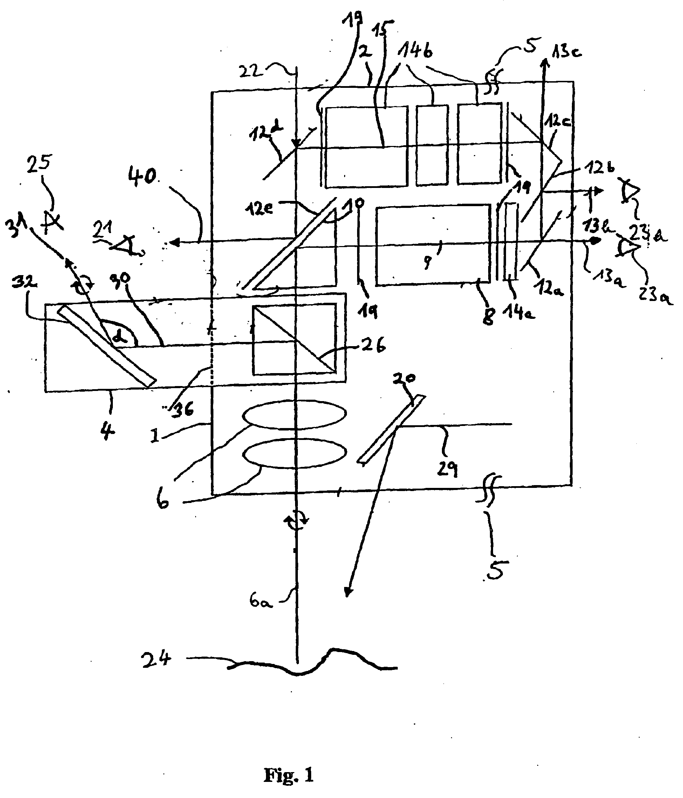

In Figur 1 ist ein schematisch dargestelltes Stereomikroskop gemäß einer bevorzugten Ausführungsform der Erfindung insgesamt mit 1 bezeichnet. Das Stereomikroskop 1 weist ein Hauptmikroskop 2 und ein Assistentenmikroskop 4 auf. Bei dem dargestellten Stereomikroskop handelt es sich insbesondere um ein ophthalmologisches Mikroskop oder eines für die Neurochirurgie.

Das Stereomikroskop 1 weist als wesentliche optische Komponenten ein Hauptobjektiv 6, ein Zoom-System 8 und wenigstens einen (nicht dargestellten) Binokulartubus mit Okularen auf. Die optische Achse 6a des Hauptobjektivs verläuft vertikal, die mittlere Achse 9 des Zoom-Systems 8 horizontal. Zwischen dem Hauptobjektiv 6 und dem Zoom-System 8 ist ein erstes Umlenkelement 10 vorgesehen. Hinter dem Zoom-System 8 sind weitere Umlenkelemente 12a bis 12e sowie optische Zusatzkomponenten 14a, 14b vorgesehen, deren Funktion bzw. Bedeutung weiter unten im einzelnen erläutert wird. Hier sei zunächst festgestellt, dass die optische Zusatzkomponente 14a auf der Achse 9 des Zoom-Systems 8 ausgebildet ist, während die optischen Zusatzkomponenten 14b auf einer hierzu parallel verlaufenden Achse 15 vorgesehen sind, wobei aus dem Zoom-System 8 austretende Strahlenbüschel nach ihrem Durchgang durch die optische Zusatzkomponente 14a an den Umlenkelementen 12a und 12c derart umgelenkt werden, dass sie durch die optischen Zusatzkomponenten 14b entlang der Achse 15 hindurchgeführt werden.

Auf den Achsen 9 und 15 sind Linsensysteme und Feldlinsen aufweisende (schematisch dargestellte) optische Elemente 19 vorgesehen, mittels derer der Strahlengang der entlang der Achse 9 des Zoom-Systems 8 bzw. der Achse 15 verlaufenden Strahlenbüschel verlängerbar ist. Die in Figur 1 dargestellten Positionen der optischen Elemente 19 sind lediglich beispielhaft gewählt. Es ist beispielsweise ebenfalls denkbar, derartige Elemente 19 zwischen den drei dargestellten optischen Zusatzkomponenten 14b auszubilden.

Mittels der optischen Elemente 19 ist entlang der Achsen 9, 15 eine mehrstufige Abbildung eines zu beobachtenden Objekts 24 in an sich bekannter Weise realisierbar. Durch das Vorsehen derartiger Elemente 19 kann die horizontale Erstreckung der Achsen 9 bzw. 15 in beliebiger Weise verlängert werden, so dass eine entsprechende Anzahl optischer Zusatzkomponenten, hier wie erwähnt mit 14a, 14b bezeichnet, wahlweise entlang dieser Achsen positionierbar ist, ohne dass hierbei die vertikale Bauhöhe des Stereomikroskops in ergonomisch ungünstiger Weise vergrößert wird. Zweckmäßigerweise kann eine weitere Trennstelle 5 vorgesehen sein, an welcher das Mikroskop zu öffnen ist und/oder optische Systeme unterschiedlicher horizontaler Erstreckung angebracht werden können.

Als hierbei vorteilhaft einsetzbare optische Zusatzkomponenten 14a, 14b seien beispielhaft eine Dateneinspiegelungseinrichtung, eine Inverter-Einrichtung, insbesondere eine SDI-Einrichtung (stereoskopischer Diagonalinverter), eine Laser-Shuttereinrichtung oder optische Teiler erwähnt. Eine Dateneinspielung kann in günstiger Weise auch unter Verwendung des Umlenkelements 12d entlang der Achse 22 vorgenommen werden, wobei dieses Umlenkelement 12d für eine derartige Dateneinspielung halbdurchlässig ausgebildet ist.

Die entlang der Achse 15 aus den optischen Zusatzkomponenten 14b austretenden Strahlenbüschel werden an dem Umlenkelement 12d senkrecht in Abwärtsrichtung umgeleitet, und an dem hierunter ausgebildeten Umlenkelement 12e wiederum in die Horizontale gelenkt. Eine bevorzugte Beobachtungsachse für einen Hauptoperateur 21 ist hier mit 40 bezeichnet. Ein durch den Hauptoperateur 21 benutzbarer Binokulartubus mit Okularen ist hier nicht im einzelnen dargestellt.
Es sei darauf hingewiesen, dass die Anzahl der optischen Zusatzkomponenten 14a, 14b je nach Bedarf wahlweise einstellbar ist. Werden für bestimmte Anwendungen entsprechende Zusatzkomponenten nicht benötigt, können an deren Stelle beispielsweise einfache Glasblöcke eingesetzt werden. Dieser Wechsel der Zusatzkomponenten kann wahlweise auch elektromotorisch erfolgen, zumindest aber mechanisch.

Mit 20 ist ein Umlenkelement einer nicht im einzelnen dargestellten Beleuchtungseinrichtung bezeichnet. Durch die Beleuchtungseinrichtung bereitgestelltes Licht mit der Beleuchtungsachse 29 wird hierbei über das Umlenkelement 20 auf das zu beobachtende Objekt 24 gerichtet.

Optional ist zwischen dem Hauptobjektiv 6 und dem ersten Umlenkelement 10 ist ein weiterer Strahlenteiler 26 vorgesehen. Der Strahlenteiler 26 teilt den HauptBeobachtungsstrahlengang entlang der Achse 6a des Hauptobjektivs 6 in zwei Teilstrahlengänge auf. Ein erster, transmittierter Teilstrahlgang entspricht dem bereits diskutierten Strahlengang, welcher nach Umlenkung durch das Umlenkelement 10 entlang der Achse 9 des Zoom-Systems 8 verläuft. Der weitere Teilstrahlengang wird vom Strahlenteiler 26 aus dem Hauptbeobachtungsstrahlengang als Assistenten-Beobachtungsstrahlengang 30 ausgespiegelt. Dieser Assistenten-Beobachtungsstrahlengang 30 wird über ein weiteres Umlenkelement 32 in einen nicht dargestellten Assistenten-Binokulartubus geführt. Das Umlenkelement 32 ermöglicht durch seine Anordnung eine Kippung und damit eine winkelveränderliche Umlenkung um den Betrag α für den Assistenten 25, wodurch eine variable Assistenten-Beobachtungsachse 31 definierbar ist.

Das Assistentenmikroskop 4 kann beispielsweise an einer mechanischen Trennstelle 36 (gestrichelt dargestellt) von dem Hauptmikroskop 2 abtrennbar sein.

Eine weitere mögliche Beobachtungsstrahlachse ist durch Ausspiegelung an den Umlenkelementen 12a, b, c realisierbar. Hier sind jeweilige Beobachtungsstrahlachsen mit 13a, b, c bezeichnet. Entsprechende Assistenten- bzw. Mitbeobachter, insbesondere monoskopische Beobachter oder Dokumentationseinrichtungen, entlang dieser Beobachtungsstrahlachsen sind mit 23a, 23b bezeichnet.

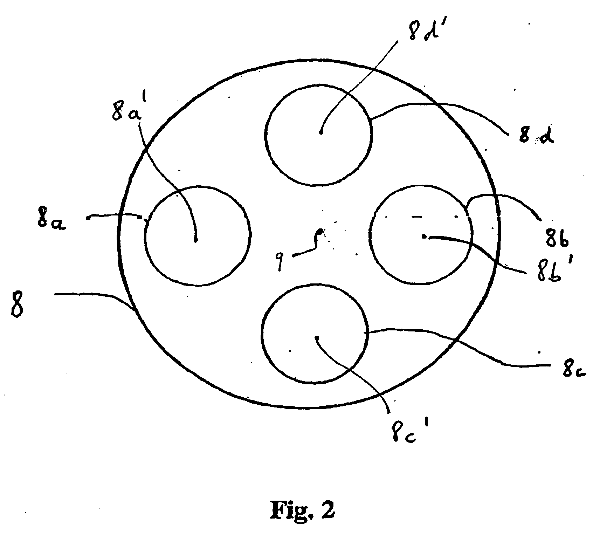

Das im Rahmen des dargestellten Stereomikroskops verwendete Zoom-System weist zweckmäßigerweise vier jeweils paarweise stereokopische Beobachtungskanäle, welche in Figur 2 mit 8a bis 8d bezeichnet sind, auf. Die optischen Achsen dieser Beobachtungskanäle sind entsprechend mit 8a' bis 8d' bezeichnet. Die Hauptbeobachtungskanäle 8a, 8b verlaufen hierbei auf einer horizontalen Ebene, d. h. im wesentlichen auf der Höhe der Achse 9 des Zoom-Systems 8. Diese Hauptbeobachtungskanäle 8a, 8b bilden zweckmäßigerweise die Beobachtungskanäle für den Hauptoperateur 21, welche nach der bereits beschriebenen Umlenkung und dem Durchlaufen durch die optischen Zusatzkomponenten 14a, 14b vom Hauptoperateur 21entlang der Beobachtungsachse 40 beobachtet werden können.

Die durch die senkrecht übereinander verlaufenden Beobachtungskanäle 8c, 8d geführten Strahlenbüschel werden vorteilhafterweise mittels des (halbdurchlässigen) Umlenkelements 12b entlang der Beobachtungsachse 13 ausgespiegelt. Es ist ebenfalls denkbar, beispielsweise das Umlenkelement 12a oder das Umlenkelement 12c derart auszubilden, dass lediglich die durch die Hauptbeobachtungskanäle 8a, 8b verlaufenden Strahlenbüschel abgelenkt werden, die durch die Beobachtungskanäle 8c, d verlaufenden Strahlenbüschel jedoch am Umlenkelement 12a vorbeigeführt werden. Bei solch einer Strahlführung können ebenfalls die Beobachtungskanäle 8a-d am Umlenkelement 12a gemeinsam reflektiert werden. Anschließend am Umlenkelement 12b werden wahlweise die Strahlenbüschel 8a, b verlustfrei vorbeigeführt und die Strahlenbüschel 8a, b vollständig in Achse 13b reflektiert. Die Strahlenbüschel 8 c, d in den Achsen 13 a, b werden bevorzugt für Dokumentationseinrichtungen oder monoskopische Mitbeobachter-Einrichtungen verwendet, da ihre Stereobasis-Linie vertikal verläuft und so - ohne weitere Umlenkung - für eine stereoskopische Betrachtung ungeeignet ist.

Bei Auskopplung der Strahlenbüschel 8c, d in der Achse 13c ist allerdings (durch Einsatz entsprechender Umlenkelemente) eine stereoskopische Beobachtung aus der Zeichenebene heraus möglich. Durch diese Maßnahme ist eine besonders lichtökonomische Teilung der Strahlengänge für Hauptoperateur und monoskopische Beobachtungs- und/oder Dokumentations-Einrichtungen durchführbar, da hier auf halbdurchlässige Spiegel verzichtet werden kann.

### Bezugszeichenliste

- 1: Stereomikroskop
- 2: Hauptmikroskop
- 4: Assistentenmikroskop
- 5: Trennstelle ,
- 6: Hauptobjektiv
- 6a: Achse des Hauptobjektivs
- 8: Zoom-System
- 8a-8d: Beobachtungskanäle des Zoom-Systems
- 8a'-8d': Achsen des Zoom-Systems
- 9: Achse des Zoom-Systems
- 10: erstes Umlenkelement
- 12a-12e: Umlenkelemente
- 13a, b, c: Beobachtungsachse
- 19: optische Elemente
- 20: Umlenkelement.
- 21: Hauptoperateur
- 22: Achse
- 23a, b: monoskopische Beobachter oder Dokumentation
- 24: Objekt
- 25: Assistenten-Beobachter
- 26: Strahlenteiler
- 29: Beleuchtungsachse
- 30: Assistenten-Strahlengang
- 31: Beobachtungsachse-Assistent
- 32: Umlenkelement
- 36: mechanische Trennstelle
- 40: Beobachtungsachse Hauptoperateur

## Patentansprüche

1. Stereomikroskop mit einem Objektiv (6) und einem dem Objektiv in Richtung des Beobachters nachgeordneten Zoom-System (8), wobei die optische Achse (6a) des Objektivs einen Winkel zur wenigstens einen optischen Achse (9, 8a', 8b', 8c', 8d') des Zoom-Systems (8) bildet, sowie einer Anzahl von Umlenkelementen (10, 12a bis 12e), mittels derer wenigstens ein aus dem Objektiv (6) in Richtung des Beobachters austretendes Beobachtungsstrahlenbüschel in das Zoom-System (8), und wenigstens ein aus dem Zoom-System (8) in Richtung des Beobachters austretendes Beobachtungsstrahlenbüschel wenigstens auf eine weitere, im wesentlichen parallel zu der wenigstens einen Achse (9, 8a', 8b', 8c', 8d') des Zoom-Systems (8) verlaufende Achse (15) umlenkbar ist,
auf den Achsen (9, 8a', 8b', 8c', 8d') des Zoom-Systems (8) und/oder auf der wenigstens einen hierzu im wesentlichen parallel verlaufenden Achse (15) wenigstens ein optisches Element (19) zur Verlängerung des Strahlengangs des wenigstens einen Beobachtungsstrahlenbüschels parallel zu der wenigstens einen optischen Achse (9, 8a', 8b', 8c', 8d') des Zoom-Systems (8) und/oder der wenigstens einen hierzu parallelen Achse (15) vorgesehen ist, **dadurch gekennzeichnet daß** das optische Element (19) Zwischenabbildungen eines zu beobachtenden Objektes (24) erzeugt.

2. Stereomikroskop nach Anspruch 1, **gekennzeichnet durch** wenigstens eine auf der wenigstens einen Achse (9, 8a', 8b', 8c', 8d') des Zoom-Systems (8) und/oder der wenigstens einen hierzu parallel verlaufenden Achse (15) vorgesehene optisch-mechanische Komponente (14a, 14b), insbesondere eine Dateneinspiegelungseinrichtung, eine Inverter-Einrichtung, eine Laser-Schuttereinrichtung oder einen optischen Teiler.

3. Stereomikroskop nach einem der vorstehenden Ansprüche, **dadurch gekennzeichnet, dass** die Komponenten (14a, b) wahlweise ein- und ausschwenkbar sind, zumindest aber optomechanisch entfernbar sind.

4. Stereomikroskop nach einem der vorstehenden Ansprüche, **dadurch gekennzeichnet, dass** die Lichtrichtungen des wenigstens einen Beobachtungsstrahlenbüschels entlang der Achse (9) des Zoom-Systems (8) und der hierzu parallel verlaufenden Achse (15) entgegengesetzt zueinander verlaufen.

5. Stereomikroskop nach einem der vorstehenden Ansprüche, **dadurch gekennzeichnet, dass** das Zoom-System (8) wenigstens drei, insbesondere vier Vergrößerungs- bzw. Beobachtungskanäle (8a, 8b, 8c, 8d) aufweist.

6. Stereomikroskop nach einem der vorstehenden Ansprüche, **dadurch gekennzeichnet, dass** die Achse (6a) des Objektivs im wesentlichen vertikal, und die wenigstens eine Achse (9, 8a', 8b', 8c', 8d') des Zoom-Systems (8) im wesentlichen horizontal verläuft.

7. Stereomikroskop nach einem der vorstehenden Ansprüche, **dadurch gekennzeichnet, dass** das Hauptobjektiv (6) zur der Achse (6a) dezentrisch angeordnet ist.

8. Stereomikroskop nach einem der vorstehenden Ansprüche, **gekennzeichnet durch** einen zwischen dem Objektiv (6) und dem Zoom-System (8) angeordneten Strahlenteiler (26).

9. Stereomikroskop nach einem der vorstehenden Ansprüche, **dadurch gekennzeichnet, dass** eine Dateneinspiegelungseinrichtung (22) vor und/oder hinter dem Zoom-System (8) ausgebildet ist.

## Claims

1. Stereomicroscope comprising an objective (6) and a zoom system (8) downstream of the objective towards a observer, the optical axis (6a) of the objective forming an angle with at least one optical axis (9, 8a', 8b', 8c', 8d') of the zoom system (8), and a plurality of deflector elements (10, 12a to 12e), by means of which at least one observation beam emerging from the objective (6) towards the observer can be guided into the zoom system (8) and at least one observation beam emerging from the zoom system (8) towards the observer can be guided at least on to one other axis (15) extending substantially parallel to the at least one axis (9, 8a', 8b', 8c', 8d') of the zoom system (8), wherein on the axes (9, 8a', 8b', 8c', 8d') of the zoom system (8) and/or on the at least one axis (15) extending substantially parallel thereto, there is provided at least one optical element (19) for extending the optical path of the at least one observation beam parallel to the at least one optical axis (9, 8a', 8b', 8c', 8d') of the zoom system (8) and/ or to the at least one axis (15) parallel thereto, **characterised in that** the optical element (19) produces intermediate images of an object (24) which is to be observed.

2. Stereomicroscope according to claim 1, **characterised by** at least one opto-mechanical component (14a, 14b) which is provided on the at least one axis (9, 8a', 8b', 8c', 8d') of the zoom system (8) and/or the at least one axis (15) extending parallel thereto, particularly a data projecting device, an inverter device, a laser shutter device or an optical splitter.

3. Stereomicroscope according to one of the preceding claims, **characterised in that** the components (14a, b) can be selectively pivoted in and out, but at least can be removed optomechanically.

4. Stereomicroscope according to one of the preceding claims, **characterised in that** the directions of the light of the at least one observation beam extend opposite to one another along the axis (9) of the zoom system (8) and the axis (15) extending parallel thereto.

5. Stereomicroscope according to one of the preceding claims, **characterised in that** the zoom system (8) comprises at least three and more particularly four magnification/observation channels (8a, 8b, 8c, 8d).

6. Stereomicroscope according to one of the preceding claims, **characterised in that** the axis (6a) of the objective extends substantially vertically and the at least one axis (9, 8a', 8b', 8c', 8d') of the zoom system (8) extends substantially horizontally.

7. Stereomicroscope according to one of the preceding claims, **characterised in that** the main objective (6) is arranged decentrically with respect to the axis (6a).

8. Stereomicroscope according to one of the preceding claims, **characterised by** a beam splitter (26) mounted between the objective (6) and the zoom system (8).

9. Stereomicroscope according to one of the preceding claims, **characterised in that** a data projecting device (22) is formed in front of and/or behind the zoom system (8).

## Revendications

1. Stéréomicroscope comportant un objectif (6) et un système de zoom (8) agencé en aval de l'objectif en direction de l'observateur, l'axe optique (6a) de l'objectif formant un angle par rapport à au moins un axe optique (9, 8a', 8b', 8c', 8d') du système de zoom (8), ainsi qu'un certain nombre d'éléments de déflexion (10, 12a à 12e) au moyen desquels au moins un faisceau de rayons d'observation sortant de l'objectif (6) en direction de l'observateur est susceptible d'être défléchi vers le système de zoom (8), et au moins un faisceau de rayons d'observation sortant du système de zoom (8) en direction de l'observateur est susceptible d'être défléchi vers au moins un autre axe (15) s'étendant sensiblement parallèlement audit au moins un axe (9, 8a', 8b', 8c', 8d') du système de zoom (8), dans lequel il est prévu, sur les axes (9, 8a', 8b', 8c', 8d') du système de zoom (8) et/ou sur ledit au moins un axe (15) s'étendant sensiblement parallèlement à ceux-ci, au moins un élément optique (19) pour prolonger la trajectoire des rayons dudit au moins un faisceau de rayons d'observation parallèlement audit au moins un axe optique (9, 8a', 8b', 8c', 8d') du système de zoom (8) et/ou audit au moins un axe (15) parallèle à celui-ci, **caractérisé en ce que** l'élément optique (19) engendre des images intermédiaires d'un objet (24) à observer.

2. Stéréomicroscope selon la revendication 1, **caractérisé par** au moins un composant optique-mécanique (14a, 14b), en particulier un dispositif d'injection de données par réflexion, un dispositif inverseur, un dispositif obturateur laser ou un subdiviseur optique, prévu sur ledit au moins un axe (9, 8a', 8b', 8c', 8d') du système de zoom (8) et/ou sur ledit au moins un axe (15) s'étendant parallèlement à celui-ci.

3. Stéréomicroscope selon l'une des revendications précédentes, **caractérisé en ce que** les composants (14a, b) sont susceptibles de pivoter au choix vers l'intérieur et vers l'extérieur, mais au moins d'être éloignés par voie opto-mécanique.

4. Stéréomicroscope selon l'une des revendications précédentes, **caractérisé en ce que** les directions de propagation dudit au moins un faisceau de rayons d'observation s'étendent en sens opposés l'une à l'autre le long de l'axe (9) du système de zoom (8) et de l'axe (15) parallèle à celui-ci.

5. Stéréomicroscope selon l'une des revendications précédentes, **caractérisé en ce que** le système de zoom (8) comprend au moins trois, en particulier quatre canaux d'agrandissement ou d'observation (8a, 8b, 8c, 8d).

6. Stéréomicroscope selon l'une des revendications précédentes, **caractérisé en ce que** l'axe (6a) de l'objectif s'étend sensiblement verticalement et ledit au moins un axe (9, 8a', 8b', 8c', 8d') du système de zoom (8) s'étend sensiblement horizontalement.

7. Stéréomicroscope selon l'une des revendications précédentes, **caractérisé en ce que** l'objectif principal (6) est agencé de façon décentrée par rapport à l'axe (6a).

8. Stéréomicroscope selon l'une des revendications précédentes, **caractérisé par** un subdiviseur de rayon (26) agencé entre l'objectif (6) et le système de zoom (8).

9. Stéréomicroscope selon l'une des revendications précédentes, **caractérisé en ce qu'**un dispositif d'injection de données par réflexion (22) est réalisé en avant et/ou en arrière du système de zoom (8).
